# EUROPEAN PATENT APPLICATION

(11) **EP 4 088 703 A1**
(43) Date of publication of application: **16.11.2022**
(21) Application number: 20912474.2
(22) Date of filing: 30.11.2020
(51) Int. Cl.: A61F 13/53, A61F 13/534, A61F 13/535

(54) **ABSORBER AND DISPOSABLE DIAPER**

(30) Priority: 10.01.2020 JP 2020002913
(71) Applicant: DSG Japan Corporation, Osaka-shi, Osaka 532-0003 (JP)
(72) Inventor: YOSHIDA, Takatoshi, Hamamatsu-shi, Shizuoka 431-2213 (JP)
(74) Representative: Berggren Oy
(86) International application number: PCT/JP2020/044567
(87) International publication number: WO 2021/140782

(57) **Abstract**

A sheet-like absorber in which an absorbent polymer is incorporated inside fiber layers formed of heat-fusible fibers and which expands by absorbing liquid, the absorber including: a channel portion that is formed over an entire length of one sheet face and extends in a sheet longitudinal direction; a first absorption layer that forms another sheet face; and plural second absorption layers that form the one sheet face and are layered an interval apart from each other in a sheet width direction on the first absorption layer, wherein the channel portion is formed by opposing surfaces of second absorption layers that are adjacent to each other and a surface of the first absorption layer, the first absorption layer and the second absorption layers are each fiber layers formed of heat-fusible fibers and have an absorbent polymer incorporated inside the fiber layers, a content of the absorbent polymer per unit area in the second absorption layers is greater than a content of the absorbent polymer per unit area in the first absorption layer, and a more durable hydrophilicity for maintaining hydrophilicity of the fibers is imparted to fibers that form portions of the second absorption layers on an opposite side from a first absorption layer side than to fibers that form portions of the second absorption layers on the first absorption layer side.

## Description

### Technical Field

The present disclosure relates to an absorber and a disposable diaper.

### Background Art

Conventionally, as an absorber for a disposable diaper, a sheet-like absorbent core that includes an absorbent polymer in fiber layers formed by liquid absorbent fibers such as pulp fibers has been used (e.g., JP-ANo. 2018-99439).

### SUMMARY OF INVENTION

### Technical Problem

The technology disclosed in JP-ANo. 2018-99439 uses the absorbent core, both sheet faces of which are flat, to spread liquid around the absorbent core and absorb the liquid, thus improving absorbency, but further improvements in absorbency are desired by the market.

In consideration of the above circumstances, it is an object of one aspect of the present disclosure to provide a sheet-like absorber whose liquid absorbency is improved by accelerating the spread of liquid in the sheet longitudinal direction and a disposable diaper using the absorber.

### Solution to Problem

An absorber of an aspect of the present disclosure is a sheet-like absorber in which an absorbent polymer is incorporated inside fiber layers formed of heat-fusible fibers and which expands by absorbing liquid, the absorber including: a channel portion that is formed over an entire length of one sheet face and extends in a sheet longitudinal direction; a first absorption layer that forms another sheet face; and plural second absorption layers that form the one sheet face and are layered an interval apart from each other in a sheet width direction on the first absorption layer, wherein the channel portion is formed by opposing surfaces of second absorption layers that are adjacent to each other and a surface of the first absorption layer, the first absorption layer and the second absorption layers are each fiber layers formed of heat-fusible fibers and have an absorbent polymer incorporated inside the fiber layers, a content of the absorbent polymer per unit area in the second absorption layers is greater than a content of the absorbent polymer per unit area in the first absorption layer, and a more durable hydrophilicity for maintaining hydrophilicity of the fibers is imparted to fibers that form portions of the second absorption layers on an opposite side from a first absorption layer side than to fibers that form portions of the second absorption layers on the first absorption layer side.

### Advantageous Effects of Invention

According to the present disclosure, there can be provided a sheet-like absorber whose liquid absorbency is improved by accelerating the spread of liquid in the sheet longitudinal direction and a disposable diaper using the absorber.

### BRIEF DESCRIPTION OF DRAWINGS

FIG. 1 is a plan view showing, in an unfolded and extended state, a disposable diaper using an absorbent of an embodiment of the disclosure.

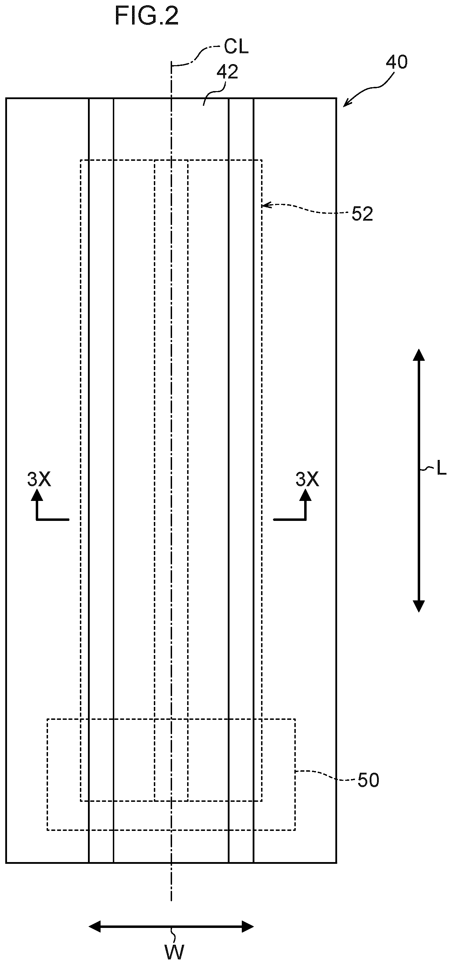

FIG. 2 is a plan view showing, in an unfolded and extended state, a front portion and a crotch portion in a central band-like region of the disposable diaper of FIG. 1.
FIG. 3 is a schematic view of a cross-section taken along line 3X-3X of FIG. 2.
FIG. 4 is a plan view showing, in an unfolded and extended state, the absorber of the embodiment of the disclosure.
FIG. 5 is a schematic view of a cross-section taken along line 5X-5X of FIG. 4.

### DESCRIPTION OF EMBODIMENT

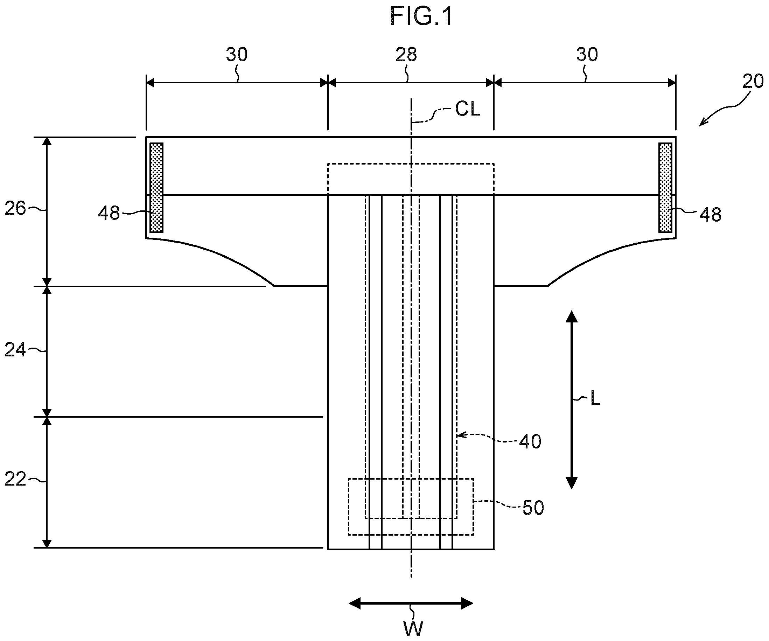

An absorber and a disposable diaper of an embodiment of the present disclosure will be described below with reference to the drawings. FIG. 1 is a plan view showing the diaper in an unfolded and extended state. It will be noted that the unfolded and extended state of the diaper is a state in which, in an unfolded state of the diaper, the diaper is extended to an extent that wrinkles that had appeared in the diaper are substantially no longer visible, and is a state in which the diaper is extended so that the dimensions of members (e.g., a later-described top sheet) configuring the diaper become lengths matching or close to the dimensions of the individual members.

### (Disposable Diaper)

A diaper 20 of this embodiment is what is called an open type disposable diaper and has a front portion 22, a crotch portion 24, and a rear portion 26 as shown in FIG. 1. The front portion 22 is a portion that becomes positioned on the front (abdominal side, front waist) of the wearer. Furthermore, the rear portion 26 is a portion that becomes positioned on the rear (back side, rear waist) of the wearer. The crotch portion 24 is a portion that becomes positioned between the front portion 22 and the rear portion 26.

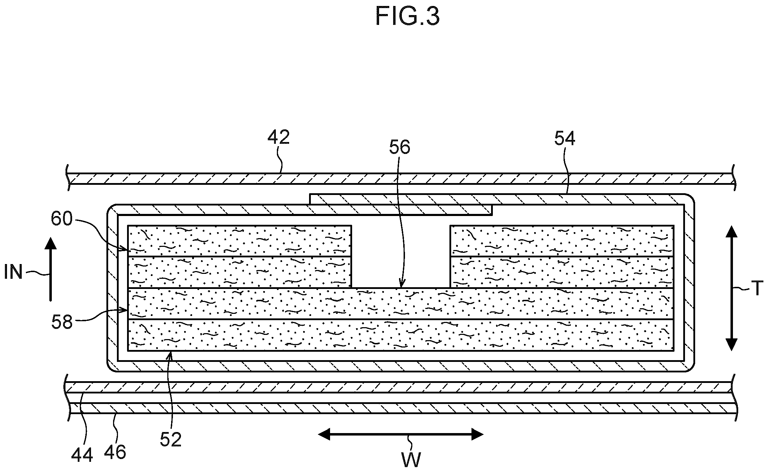

Hereinafter, directions of the diaper 20 will be defined as shown in FIG. 1. The direction heading from the front portion 22 to the rear portion 26 will be defined as a longitudinal direction of the diaper 20 (the direction indicated by arrow L in FIG. 1), and the direction orthogonal to the longitudinal direction of the diaper 20 will be defined as a width direction of the diaper 20 (the direction indicated by arrow W in FIG. 1). Furthermore, as shown in FIG. 3, the direction orthogonal to the longitudinal direction and the width direction of the diaper 20 will be defined as a thickness direction of the diaper 20, the side adjacent to the skin of the wearer in the thickness direction of the diaper 20 will be defined as a skin side (the side indicated by arrow IN in FIG. 3), and the opposite side thereof will be defined as a non-skin side.

The diaper 20 has a central band-like region 28 and side flaps 30.

The central band-like region 28 is a band-like region positioned in the central portion in the width direction of the diaper 20 (see FIG. 1) and ranges over the front portion 22, the crotch portion 24, and the rear portion 26. The central band-like region 28 is a portion of the diaper 20 that absorbs and holds liquid such as urine excreted by the wearer. The central band-like region 28 mainly has an absorbent body 40, a top sheet 42 (an example of a skin-side sheet member in the present disclosure), a leakproof sheet 44 (an example of a non-skin-side sheet member in the present disclosure), and a back sheet 46 (see FIG. 3).

The absorbent body 40 is a member that can absorb excreta such as urine. The absorbent body 40 will be described in detail later.

The top sheet 42 is a member that is disposed on the skin side of the absorbent body 40 and is permeable to liquid.

The leakproof sheet 44 is a member that is disposed on the non-skin side of the absorbent body 40 and is impermeable to liquid but permeable to moisture.

The back sheet 46 is a member (exterior sheet) configuring the exterior on the non-skin side of the diaper 20 and is configured by nonwoven fabric, for example. The leakproof sheet 44 is disposed between the back sheet 46 and the absorbent body 40 (see FIG. 3).

In at least the crotch portion 24 of the central band-like region 28, rubber threads serving as an example of a pair of leg-encircling elastic members (not shown in the drawings) that can extend and contract in the longitudinal direction of the diaper 20 are provided between the absorbent body 40 and the back sheet 46. The leg-encircling elastic members impart elasticity to the central band-like region 28 of the crotch portion 24. Contractive force along the longitudinal direction is generated with respect to the crotch portion 24 of the central band-like region 28 by the leg-encircling elastic members, whereby the fit of the diaper 20 improves and it becomes easier to inhibit excreta from leaking to the outside of the diaper 20.

The front portion 22 of the central band-like region 28 is provided with a target tape 50 (see FIG. 1). The target tape 50 is disposed in the front portion 22 on the non-skin side of the back sheet 46. The target tape 50 is a member that can engage with engaging portions of fastening tapes 48 and is made of nonwoven fabric, for example.

The side flaps 30 are portions positioned on both width direction sides of at least the rear portion 26 of the central band-like region 28 (see FIG. 1). Both end sides of these side flaps 30 in the width direction of the diaper 20 are provided with fastening tapes 48 that have engaging portions. These fastening tapes 48 are, for example, hook-and-loop fasteners, and can be stuck to the target tape 50 provided in the front portion 22 of the central band-like region 28.

### (Absorber)

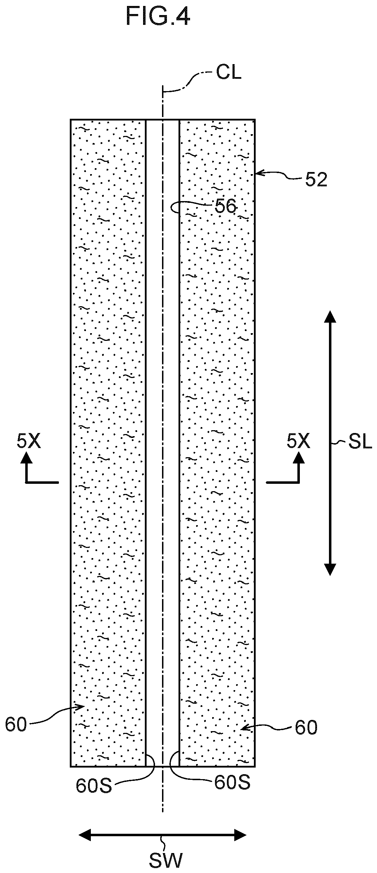

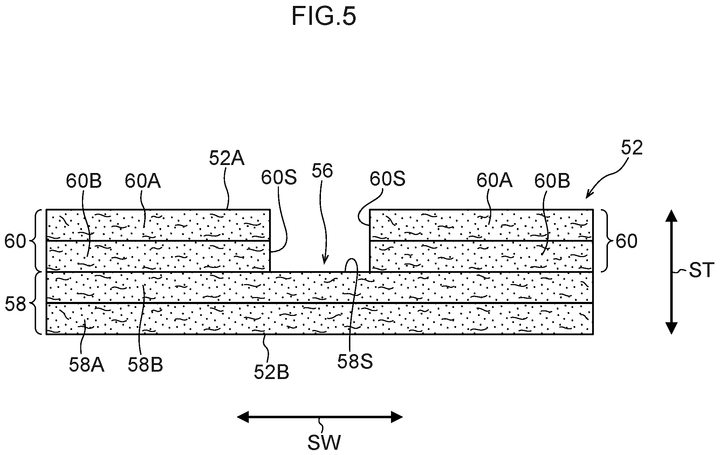

As shown in FIG. 5, the absorbent body 40 is configured to include an absorber 52 and a wrap sheet 54 that wraps the absorber 52 and is permeable to liquid. It will be noted that hereinafter the longitudinal direction of the absorber 52 will be described as a sheet longitudinal direction (the direction indicated by arrow SL in FIG. 4), the width direction of the absorber 52 will be described as a sheet width direction (the direction indicated by arrow SW in FIG. 4), and the thickness direction of the absorber 52 will be described as a sheet thickness direction (the direction indicated by arrow ST in FIG. 5). Furthermore, the sheet longitudinal direction, the sheet width direction, and the sheet thickness direction of the absorber 52 correspond to the longitudinal direction, the width direction, and the thickness direction of the diaper 20, respectively.

The absorber 52 is a sheet-like absorber that includes an absorbent polymer and expands by absorbing liquid. In one sheet face 52A of the absorber 52 is formed a channel portion 56 that extends along the sheet longitudinal direction. The channel portion 56 is formed over an entire length of the absorber 52. That is, the channel portion 56 extends continuously along the sheet longitudinal direction from one end in the sheet longitudinal direction of the absorber 52 to the other end, and opens at both ends in the sheet longitudinal direction of the absorber 52. Specifically, the absorber 52 has a first absorption layer 58 that forms another sheet face 52B and plural second absorption layers 60 that form the one sheet face 52A and are layered an interval apart from each other in the sheet width direction on the first absorption layer 58, and the channel portion 56 is formed by opposing surfaces 60S of the second absorption layers 60 that are adjacent to each other and a surface 58S of the first absorption layer 58. It will be noted that in this embodiment, the second absorption layers 60 are disposed as a pair on both sheet width direction sides of a center line CL in the sheet width direction of the first absorption layer 58. For this reason, the channel portion 56 is formed on the center line CL of the first absorption layer 58 and along the center line CL. Furthermore, in this embodiment, the pair of second absorption layers 60 have the same dimensions and shape.

Furthermore, as shown in FIG. 5, the thicknesses of the first absorption layer 58 and the second absorption layers 60 configuring the absorber 52 are substantially the same thickness.

The first absorption layer 58 and the second absorption layers 60 configuring the absorber 52 are each fiber layers including an absorbent polymer. Specifically, the first absorption layer 58 and the second absorption layers 60 are fiber layers in which heat-fusible fibers (e.g., polyolefin-based fibers such as polyethylene fibers and polypropylene fibers) are molded into a predetermined shape, and an absorbent polymer (what is called an SAP) or the like is incorporated inside the fiber layers. It will be noted that although in this embodiment an air-through nonwoven fabric using polyolefin-based fibers is used as the fiber layers, the present disclosure is not limited to this configuration.

Furthermore, in the absorber 52, a content C2 of the absorbent polymer per unit area in the second absorption layers 60 is greater than a content C1 of the absorbent polymer per unit area in the first absorption layer 58. Specifically, the content C2 is in the range of 150% to 500%, and preferably in the range of 250% to 400%, of the content C1.

Furthermore, a more durable hydrophilicity for maintaining hydrophilicity of the fibers is imparted to fibers that form portions 60A of the second absorption layers 60 on an opposite side from a first absorption layer 58 side than to fibers that form portions 60B of the second absorption layers 60 on the first absorption layer 58 side. It will be noted that known methods can be used to impart hydrophilicity to fibers. It will be noted that in this embodiment a known hydrophilic fiber treatment agent (as an example, the treatment agent described in Japanese Patent No. 3,362,348) is used to impart the durable hydrophilicity to fibers that form the portions 60A of the second absorption layers 60. By contrast, a common hydrophilicity is imparted to fibers that form the portions 60B of the second absorption layers 60. In this embodiment, the second absorption layers 60 have a two-layer structure, and the second absorption layers 60 are formed by layering fiber layers (as an example, air-through nonwoven fabrics) in which the portions 60A and the portions 60B have been subjected to different treatments.

A more durable hydrophilicity for maintaining the hydrophilicity of the fibers is imparted to fibers that form a portion 58A of the first absorption layer 58 on the opposite side from the second absorption layers 60 side than to fibers that form a portion 58B of the first absorption layer 58 on the second absorption layers 60 side. It will be noted that in this embodiment, in the same way as with the portions 60A of the second absorption layers 60, a known hydrophilic fiber treatment agent is used to impart the durable hydrophilicity to fibers that form the portion 58Aof the first absorption layer 58. By contrast, a common hydrophilicity is imparted to fibers that form the portion 58B of the first absorption layer 58. In this embodiment, the first absorption layer 58 has a two-layer structure, and the first absorption layer 58 is formed by layering fiber layers (as an example, air-through nonwoven fabrics) in which the portion 58A and the portion 58B have been subjected to different treatments.

The absorbent body 40 having the absorber 52 configured in this way is disposed between the top sheet 42 and the leakproof sheet 44 so that the one sheet face 52A of the absorber 52 in which the channel portion 56 is formed is positioned on the top sheet 42 side and the other sheet face 52B of the absorber 52 is positioned on the leakproof sheet 44 side.

Next, the action of this embodiment will be described.

In the absorber 52 of this embodiment, the channel portion 56 that extends in the sheet longitudinal direction is formed in the one sheet face 52A. Here, when liquid flows from the one sheet face 52A side into the absorber 52, some of the inflowing liquid is absorbed by the absorber 52 as it moves through the channel portion 56 in the sheet longitudinal direction. In this way, in the absorber 52, the channel portion 56 is formed in the one sheet face 52A (the top sheet 42 side), so compared, for example, to a configuration where the one sheet face is flat, the spread of the liquid in the sheet longitudinal direction can be accelerated. Because of this, the absorbency of the absorber 52 improves.

Furthermore, in the absorber 52, the channel portion 56 is formed over the entire length, so compared, for example, to a configuration where the channel portion 56 is intermittently formed, the liquid can continuously move to both ends in the sheet longitudinal direction of the absorber 52 and the liquid can be spread over a wider range. Because of this, the spread of the liquid in the sheet longitudinal direction can be further accelerated.

Furthermore, in the absorber 52, the plural second absorption layers 60 are layered an interval apart from each other in the sheet width direction on the first absorption layer 58, and the channel portion 56 is formed by the opposing surfaces 60S of the second absorption layers 60 that are adjacent to each other and the surface 58S of the first absorption layer 58. In this way, in the absorber 52, the channel portion 56 is formed by layering the plural second absorption layers 60 on the first absorption layer 58, so compared, for example, to an absorber where the channel portion 56 is processed in a single absorption layer, manufacturing becomes easier.

Furthermore, in the absorber 52, the content C2 in the second absorption layers 60 is greater than the content C1 in the first absorption layer, so compared, for example, to an absorber where the contents of the absorbent polymer per unit area in the first absorption layer 58 and the second absorption layers 60 are the same, the amount of expansion in the thickness direction of the second absorption layers 60 during liquid absorption becomes larger and the channel depth of the channel portion 56 becomes deeper. In this way, in the absorber 52, the channel depth of the channel portion 56 becomes deeper by absorbing liquid, so more liquid can be spread through the channel portion 56 in the sheet longitudinal direction and liquid absorbency improves.

Furthermore, in the absorber 52, a durable hydrophilicity is imparted to fibers that form the portions 60A of the second absorption layers 60, and a common hydrophilicity (a hydrophilicity that, compared to the durable hydrophilicity, does not have the effect of maintaining the hydrophilicity of the fibers) is imparted to fibers that form the portions 60B of the second absorption layers.

Here, during the initial stage of liquid absorption, the absorber 52 mainly absorbs liquid with the portion 58B of the first absorption layer 58 to which the common hydrophilicity is imparted and the portions 60B of the second absorption layers 60 to which the common hydrophilicity is imparted. Then, from the intermediate stage of liquid absorption on, even if absorbency drops in association with a drop in the hydrophilicity of the portions of the second absorption layers 60 to which the common hydrophilicity is imparted, the liquid can be absorbed by the portions 60A of the second absorption layers 60 to which the durable hydrophilicity is imparted, so an effect of inhibiting backtracking of the liquid can be expected.

Furthermore, in the absorber 52, the second absorption layers 60 are disposed as a pair on both sheet width direction sides of the center line CL of the first absorption layer 58, so compared, for example, to a configuration where three or more second absorption layers are formed, the number of parts of the members configuring the second absorption layers 60 can be reduced. In particular, in this embodiment, because the pair of second absorption layers 60 have the same dimensions and shape, the number of parts can be further reduced.

As described above, the diaper 20 uses the absorber 52 whose liquid absorbency is improved by accelerating the spread of liquid in the sheet longitudinal direction, so wearer comfort improves.

In the absorber 52 of the above embodiment, the single channel portion 56 is formed along the center line CL of the first absorption layer 58, but the present disclosure is not limited to this configuration. For example, a plurality of the channel portions 56 may also be formed in the first absorption layer 58. Furthermore, the cross-sectional shapes of each of the channels in the case of forming a plurality of the channel portions may be the same or different. It will be noted that although in this embodiment the cross-sectional shape of the channel portion 56 is substantially quadrilateral, the present disclosure is not limited to this configuration. For example, the shape of the channel portion 56 may also be substantially V-shaped, substantially trapezoidal, or substantially arc-shaped. Furthermore, the channel portion 56 may also be formed in the shape of a zigzag, the shape of a sine wave, or the shape of a square wave along the center line CL of the first absorption layer 58.

In the absorber 52 of the above embodiment, the channel portion 56 is formed over the entire length, but the present disclosure is not limited to this configuration. For example, the channel portion 56 may also be intermittently formed in the sheet longitudinal direction. For example, the channel portion 56 may also be continuous, leaving only the vicinities of both ends in the sheet longitudinal direction of the absorber 52.

In the absorber 52 of the above embodiment, a durable hydrophilicity is imparted to fibers of the portion 58A of the first absorption layer 58, and a common hydrophilicity is imparted to fibers of the portion 58B, but the present disclosure is not limited to this configuration. For example, a durable hydrophilicity may also be imparted to both the fibers of the portion 58Aand the fibers of the portion 58B of the first absorption layer 58.

Furthermore, in the absorber 52 of the above embodiment, the first absorption layer 58 and the second absorption layers 60 each have a two-layer structure, but the present disclosure is not limited to this configuration. For example, the first absorption layer 58 and the second absorption layers 60 may also each have a single-layer structure or a structure of three or more layers. It will be noted that in a case where the second absorption layers 60 have a structure of three or more layers, it is preferable to impart a durable hydrophilicity to fibers configuring the uppermost layers.

In the absorber 52 of the above embodiment, the thicknesses of the first absorption layer 58 and the second absorption layers 60 are substantially the same, but the present disclosure is not limited to this configuration. For example, the thicknesses of the first absorption layer 58 and the second absorption layers 60 may also be different thicknesses.

The absorber 52 of the above embodiment is used in the open type (taped type) diaper 20, but the present disclosure is not limited to this configuration. For example, the absorber 52 may also be used in a pants type disposable diaper, and may also be used in other disposable diapers in which a taped type and a pants type are combined.

An embodiment of the present disclosure has been described above, but the disclosure is not limited to what is described above and can of course be modified and implemented in various ways, in addition to what is described above, in a range that does not depart from the spirit thereof.

It will be noted that the following supplementary notes are further disclosed in relation to the above description.

### (Supplementary Note 1)

An absorber of supplementary note 1 is a sheet-like absorber that includes an absorbent polymer and expands by absorbing liquid, and a channel portion that extends in a sheet longitudinal direction is formed in one sheet face of the absorber.

In the absorber of supplementary note 1, the channel portion that extends in the sheet longitudinal direction is formed in the one sheet face. Here, when liquid flows from the one sheet face side into the absorber, some of the inflowing liquid is absorbed by the absorber as it moves through the channel portion in the sheet longitudinal direction. In this way, in this absorber, the channel portion is formed in the one sheet face, so compared, for example, to a configuration where the one sheet face is flat, the spread of the liquid in the sheet longitudinal direction can be accelerated. Because of this, the absorbency of the absorber improves.

### (Supplementary Note 2)

An absorber of supplementary note 2 is the absorber of supplementary note 1, wherein the channel portion is formed over the entire length.

In the absorber of supplementary note 2, the channel portion is formed over the entire length, so compared, for example, to a configuration where the channel portion is intermittently formed, the liquid can continuously move to both ends in the sheet longitudinal direction of the absorber and the liquid can be spread over a wider range. Because of this, the spread of the liquid in the sheet longitudinal direction can be further accelerated.

### (Supplementary Note 3)

An absorber of supplementary note 3 is the absorber of supplementary note 2, wherein the absorber has a first absorption layer that forms another sheet face and a plurality of second absorption layers that form the one sheet face and are layered an interval apart from each other in a sheet width direction on the first absorption layer, and the channel portion is formed by opposing surfaces of second absorption layers that are adjacent to each other and a surface of the first absorption layer.

In the absorber of supplementary note 3, the plural second absorption layers are layered an interval apart from each other in the sheet width direction on the first absorption layer, and the channel portion is formed by the opposing surfaces of second absorption layers that are adjacent to each other and the surface of the first absorption layer. In this way, in this absorber, the channel portion is formed by layering the plural second absorption layers on the first absorption layer, so compared, for example, to an absorber where the channel portion is processed in a single absorption layer, manufacturing becomes easier.

### (Supplementary Note 4)

An absorber of supplementary note 4 is the absorber of supplementary note 3, wherein the first absorption layer and the second absorption layers are each fiber layers that include an absorbent polymer, and a content of the absorbent polymer per unit area in the second absorption layers is greater than a content of the absorbent polymer per unit area in the first absorption layer.

In the absorber of supplementary note 4, the content of the absorbent polymer per unit area in the second absorption layers is greater than the content of the absorbent polymer per unit area in the first absorption layer, so compared, for example, to an absorber where the contents of the absorbent polymer per unit area in the first absorption layer and the second absorption layers are the same, the amount of expansion in the thickness direction of the second absorption layers during liquid absorption becomes larger and the channel depth of the channel portion becomes deeper. In this way, in this absorber, the channel depth of the channel portion becomes deeper by absorbing liquid, so more liquid can be spread through the channel portion in the sheet longitudinal direction and liquid absorbency improves.

### (Supplementary Note 5)

An absorber of supplementary note 5 is the absorber of supplementary note 4, wherein a more durable hydrophilicity for maintaining hydrophilicity of the fibers is imparted to fibers that form portions of the second absorption layers on an opposite side from a first absorption layer side than to fibers that form portions of the second absorption layers on the first absorption layer side.

In the absorber of the fifth aspect, a durable hydrophilicity is imparted to fibers that form the portions of the second absorption layers on the opposite side from the first absorption layer side, and a common hydrophilicity (a hydrophilicity that, compared to the durable hydrophilicity, does not have the effect of maintaining the hydrophilicity of the fibers) is imparted to fibers that form the portions of the second absorption layers on the first absorption layer side.

Here, during the initial stage of liquid absorption, the absorber mainly absorbs liquid with the first absorption layer and the portions of the second absorption layers to which the common hydrophilicity is imparted. Then, from the intermediate stage of liquid absorption on, even if absorbency drops in association with a drop in the hydrophilicity of the portions of the second absorption layers to which the common hydrophilicity is imparted, the liquid can be absorbed by the portions of the second absorption layers to which the durable hydrophilicity is imparted, so an effect of inhibiting backtracking of the liquid can be expected.

### (Supplementary Note 6)

An absorber of supplementary note 6 is the absorber of any one of supplementary note 3 to supplementary note 5, wherein the second absorption layers are disposed as a pair on both sheet width direction sides of a center line in the sheet width direction of the first absorption layer.

In the absorber of supplementary note 6, the second absorption layers are disposed as a pair on both sheet width direction sides of the center line in the sheet width direction of the first absorption layer, so compared, for example, to a configuration where three or more second absorption layers are formed, the number of parts of the members configuring the second absorption layers can be reduced.

### (Supplementary Note 7)

A disposable diaper of supplementary note 7 is a disposable diaper using the absorber of any one of supplementary note 1 to supplementary note 6, the disposable diaper including: an absorbent body configured to include the absorber and a wrap sheet that wraps the absorber and is permeable to liquid; a skin-side sheet member that is disposed on a skin side of the absorbent body and is permeable to liquid; and a non-skin-side sheet member that is disposed on the opposite side of the skin side of the absorbent body and is impermeable to liquid but permeable to moisture, wherein the one sheet face of the absorber in which the channel portion is formed is positioned on the non-skin-side sheet member and the other sheet face of the absorber is positioned on the non-skin-side sheet member side.

The disposable diaper of supplementary note 7 uses the absorber whose liquid absorbency is improved by accelerating the spread of liquid in the sheet longitudinal direction, so wearer comfort improves.

It will be noted that the disclosure of Japanese Patent Application 2020-002913 filed on January 10, 2020, is incorporated in its entirety by reference herein.

All documents, patent applications, and technical standards mentioned in this specification are incorporated by reference herein to the same extent as if each individual document, patent application, or technical standard were specifically and individually indicated to be incorporated by reference.

## Claims

1. A sheet-like absorber in which an absorbent polymer is incorporated inside fiber layers formed of heat-fusible fibers and which expands by absorbing liquid, the absorber comprising:
a channel portion that is formed over an entire length of one sheet face and extends in a sheet longitudinal direction;
a first absorption layer that forms another sheet face; and
a plurality of second absorption layers that form the one sheet face and that are layered an interval apart from each other in a sheet width direction on the first absorption layer,
wherein:
the channel portion is formed by opposing surfaces of second absorption layers that are adjacent to each other and a surface of the first absorption layer,
the first absorption layer and the second absorption layers are each fiber layers formed of heat-fusible fibers and have an absorbent polymer incorporated inside the fiber layers,
a content of the absorbent polymer per unit area in the second absorption layers is greater than a content of the absorbent polymer per unit area in the first absorption layer, and
a more durable hydrophilicity for maintaining hydrophilicity of the fibers is imparted to fibers that form portions of the second absorption layers on an opposite side from a first absorption layer side than to fibers that form portions of the second absorption layers on the first absorption layer side.

2. The absorber of claim 1, wherein a more durable hydrophilicity for maintaining the hydrophilicity of the fibers is imparted to fibers that form a portion of the first absorption layer on an opposite side from a second absorption layers side than to fibers that form a portion of the first absorption layer on the second absorption layers side.

3. The absorber of claim 1 or claim 2, wherein the second absorption layers are disposed as a pair on both sheet width direction sides of a center line in the sheet width direction of the first absorption layer.

4. A disposable diaper using the absorber of any one of claim 1 to claim 3, the disposable diaper comprising:
an absorbent body configured to include the absorber and a wrap sheet that wraps the absorber and is permeable to liquid;
a skin-side sheet member that is disposed on a skin side of the absorbent body and is permeable to liquid; and
a non-skin-side sheet member that is disposed on an opposite side from the skin side of the absorbent body and is impermeable to liquid but permeable to moisture,
wherein the one sheet face of the absorber in which the channel portion is formed is positioned on the non-skin-side sheet member side and the other sheet face of the absorber is positioned on the non-skin-side sheet member side.
